# EUROPÄISCHE PATENTSCHRIFT

(11) **EP 2 688 534 B1**
(45) Veröffentlichungstag und Bekanntmachung des Hinweises auf die Patenterteilung: **04.03.2015**
(21) Anmeldenummer: 12718574.2
(22) Anmeldetag: 21.03.2012
(51) Int. Cl.: A61F 5/30, A61F 13/00, A61B 17/132, A61F 5/40

(54) **EINRICHTUNG ZUM ANDRÜCKEN EINES KOMPRESSORIUMS**
DEVICE FOR PRESSING A COMPRESSION DEVICE ON
DISPOSITIF DE COMPRESSION D'UN APPAREIL DE COMPRESSION

(30) Priorität: 22.03.2011 DE 102011014602
(43) Veröffentlichungstag der Anmeldung: 29.01.2014
(73) Patentinhaber: Östreicher, Ulrich, 37276 Meinhard (DE)
(72) Erfinder: Östreicher, Ulrich, 37276 Meinhard (DE)
(74) Vertreter: Gittinger, Andreas
(86) Internationale Anmeldenummer: PCT/DE2012/000295
(87) Internationale Veröffentlichungsnummer: WO 2012/126460

(56) Entgegenhaltungen:
- WO-A1-03/099143
- GB-A- 2 205 243
- GB-A- 191 311 947
- US-A- 5 695 520
- US-A1- 2007 088 386

## Beschreibung

Die Erfindung betrifft eine Einrichtung zum Andrücken eines Kompressoriums mit einer Schnalle, durch die ein Verband führbar ist.

Aus dem Stand der Technik ist allgemein ein Druckverband bekannt, bei dem das Kompressorium oberhalb einer abzudrückenden Wunde positioniert wird und der Druckverband mehrfach um das Kompressorium und das die Wunde aufweisende Körperteil herumgeschlungen wird, um somit das Kompressorium mit einem möglichst hohen Druck gegen die Wunde zu drücken.

Die Erfindung hat die Aufgabe, eine Einrichtung der eingangs genannten Art dahingehend zu verbessern, das zukünftig eine zuzudrückende Wunde mit wenigen Handgriffen und minimalem Kraftaufwand zugedrückt werden kann.

Die Erfindung löst die gestellte Aufgabe mit einer Einrichtung der eingangs genannten Art gemäß Anspruch 1. Ein Verband kann somit um mindestens zwei der drei Stege herumgeführt werden, um ein Kompressorium mit wenigen Handgriffen gegen eine zuzudrückende Wunde zu pressen. Der Verband muss also nicht mehr wie seither einmal oder mehrfach um ein die zuzudrückende Wunde aufweisendes Körperteil herumgeschlungen werden, so dass das Anlegen des Verbands und ein Stoppen einer Blutung der Wunde in kurzer Zeit mit wenigen Handgriffen geschehen kann. Da der Verband um die Stege herumgeführt wird, kann er mit minimalem Kraftaufwand gespannt werden, wodurch das Kompressorium mit hohem Druck gegen die Wunde, die eine Punktionsstelle sein kann, gedrückt wird und somit die Blutung schnell gestoppt wird. Die Einrichtung kann insbesondere zum Zudrücken einer Punktionsstelle einer Arterie im Bereich eines Gelenks, insbesondere eines Handgelenks oder eines Gelenks im Bereich einer Ellenbogenbeuge, verwendet werden.

Um das Kompressorium an einer bestimmten Stelle genau positionieren zu können, kann es zwischen zwei benachbarten Stegen angeordnet werden. Die Stege definieren die Position des Kompressorium. Die Stege können formschlüssig in im Kompressorium vorgesehene Nuten oder im Kompressorium vorgesehene Aussparungen eingreifen. In einer besonderen Ausführungsform können die Nuten und die Stege schwalbenschwanzartig ausgebildet sein.

Damit sich die Einrichtung möglichst gut an die Form des die Wunde aufweisenden Körperteils anschmiegen kann, kann die Schnalle eine gewisse Flexibilität oder an den Querstegen mindestens ein Scharnier aufweisen. Somit wird die Anpressung des Kompressoriums an die Wunde zusätzlich verbessert.

Zur Befestigung des Verbandes kann die Schnalle an einem der beiden äußeren Stege eine Platte aufweisen. Der Verband kann an der Platte beispielsweise festgeklebt oder angenäht sein.

Damit sich der gespannte Verband nicht entlang seiner Breite wurstartig zusammenschiebt, kann einer der äußeren Stege eine der Schnalleininnenseite zugewandte konvexe Wölbung aufweisen.

Das Kompressorium kann als eine Lupe ausgebildet sein oder eine Lupe aufweisen. Dadurch kann eine Hautoberfläche vergrößert werden, um die Wunde besser erkennen zu können. Die ist besonders dann nützlich, wenn die Wunde die Punktionsstelle ist. Zweckmäßigerweise kann das Kompressorium aus einem durchsichtigen Material gefertigt sein.

Das Kompressorium kann aus einem elastisch verformbaren Material hergestellt sein, wobei das Kompressorium die Gestalt eines seiner Länge nach halbierten Zylinders aufweisen kann. Somit kann das Kompressorium mit einer gewölbten Oberfläche sehr genau auf die zuzudrückende Wunde aufgelegt werden, wobei es beim Spannen des Verbandes in Richtung der Dicke des Kompressorium zusammengedrückt wird, so dass der erzeugte große Druck gleichmäßig auf eine größere Fläche verteilt wird. Dies ist besonders dann sinnvoll, wenn die Wunde die Punktionsstelle in einer Arterie ist. Dann wird auf die Arterie ein möglichst großer Druck gleichmäßig ausgeübt.

Die Erfindung betrifft außerdem einen Verband, der erfindungsgemäß die Einrichtung nach einem der Ansprüche 1 bis 7 aufweist.

Der Verband kann einenends eine geschlossene Schlaufe aufweisen, die um einen der mindestens drei Stege herumgeführt ist, und er kann anderenends um einen weiteren der mindestens drei Stege herumgeführt sein und einen Verschluss aufweisen. Somit lässt sich der Verband mit wenigen Handgriffen anlegen und die Wunde und minimalem Kraftaufwand zudrücken. Der Verschluss kann ein Klettverschluss oder ein Schnappverschluss, bei dem eine Zunge in eine Öffnung einrastet, sein. Alternativ kann der Verschluss auch unmittelbar an der Schnalle angeordnet sein.

Zweckmäßigerweise kann der Verband einstückig ausgeführt sein. Es müssen also nicht mehrere Abschnitte zur Herstellung des Verbandes zusammengefügt werden. Dadurch kann der Verband preiswert hergestellt werden.

Nachfolgend werden verschiedene Ausführungsbeispiele einer erfindungsgemäßen Einrichtung anhand der beiliegenden Zeichnungen näher erläutert.

Im Einzelnen zeigen:
- Fig. 1: eine Schnittansicht durch eine Schnalle;
- Fig. 2: eine Vorderansicht auf eine erste Ausführungsform der Einrichtung;
- Fig. 3: eine Vorderansicht auf eine zweite Ausführungsform der Einrichtung und eine zweite Ausführungsform der Schnalle;
- Fig. 4: eine Schnittansicht durch die im Einsatz befindliche Einrichtung aus Fig. 3;
- Fig. 5: eine Draufsicht auf eine dritte Ausführungsform der Schnalle;
- Fig. 6: eine Schnittansicht durch die im Einsatz befindliche Einrichtung aus Fig. 5;
- Fig. 7: eine Draufsicht auf eine vierte Ausführungsform der Schnalle;
- Fig. 8: eine Schnittansicht durch die Schnalle aus Fig. 7;
- Fig. 9: eine perspektivische Draufsicht auf die Einrichtung mit einer fünften Ausführungsform der Schnalle;
- Fig. 10: eine Schnittansicht durch die im Einsatz befindliche Einrichtung aus Fig. 9;
- Fig. 11: eine perspektivische Draufsicht auf eine sechste Ausführungsform der Schnalle;
- Fig. 12: eine perspektivische Draufsicht auf ein Kompressorium;
- Fig. 13: eine Schnittansicht durch die Einrichtung mit der Schnalle aus Fig. 11;
- Fig. 14: eine Schnittansicht durch die Einrichtung mit einer formschlüssigen Verbindung zwischen der Schnalle und dem Kompressorium;
- Fig. 15: eine perspektivische Draufsicht einer siebten Ausführungsform der Schnalle;
- Fig. 16: eine perspektivische Draufsicht auf eine zweite Ausführungsform des Kompressoriums;
- Fig. 17: eine Vorderansicht durch eine achte Ausführungsform der Schnalle;
- Fig. 18: eine Vorderansicht durch eine dritte Ausführungsform des Kompressorium.

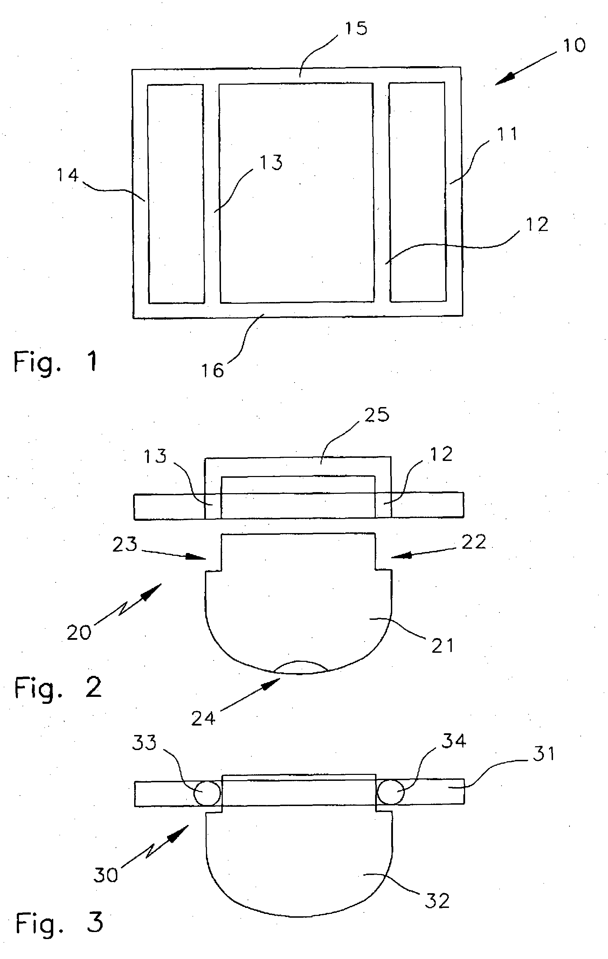

Die Fign. 1 und 2 zeigen eine Schnalle 10 einer Einrichtung 20 Spannen eines Verbandes und zum Andrücken eines Kompressoriums 21. Die Schnalle 10 ist mit Stegen 11, 12, 13 und 14 versehen, welche Querstege 15 und 16 miteinander verbinden. Um die Stege 11 und 14 kann der Verband herumgeführt werden.

Das Kompressorium 21 kann zwischen den Stegen 12 und 13 angeordnet werden. Das Kompressorium 21 weist einen pilzartigen Querschnitt mit Aussparungen 22 und 23 auf. In die Aussparungen 22 und 23 können die Stege 12 und 13 eingreifen. Auf diese Weise kann das Kompressorium 21 in einer genau definierten Position oberhalb einer Wunde, die eine Punktionsstelle sein kann, platziert werden, um diese beim Spannen des Verbandes, wobei das Kompressorium 21 gegen die Wunde gedrückt wird, zuzudrücken. Ein Bügel 25, wie oberhalb der Stege 12 und 13 angeordnet ist und diese miteinander verbindet, verändert, dass das Kompressorium 21 nach oben heraus gedrückt werden kann. Der Bügel 25 kann sich über die gesamte Länge der Stege 12 und 13 erstrecken oder über eine Teillänge der Stege 12 und 13.

Das Kompressorium 21 ist mit einer Lupe 24 ausgerüstet. Dadurch kann eine Hautoberfläche vergrößert werden, um eine verhältnismäßig kleine Punktionsstelle zu vergrößern und somit das Kompressorium exakt an der erforderlichen Stelle zu positionieren.

Eine Einrichtung 30 mit einer Schnalle 31 und einem Kompressorium 32 (siehe Fig. 3) weist an ihrer Schnalle 31 Scharniere 33 und 34 auf. Das Kompressorium 32 ist zwischen den Scharnieren 33 und 34 angeordnet. Die Scharniere 33 und 34 erfüllen somit auch die Funktion der Stege. Somit können sich die Schnalle 31 respektive die Einrichtung 30 gut an die Form eines die Wunde aufweisenden Körperteils anschmiegen, wodurch die Anpressung des Kompressorium 32 an die Wunde zusätzlich verbessert wird. Wegen der Anschmiegsamkeit der Einrichtung 30 kann ein und dieselbe Einrichtung 30 auch bei Personen unterschiedlicher Größe angelegt werden. Deshalb eignet sich die Einrichtung 30 besonders gut, um einer Arterie im Bereich eines Handgelenks oder eines Gelenks im Bereich einer Ellenbogenbeuge zuzudrücken.

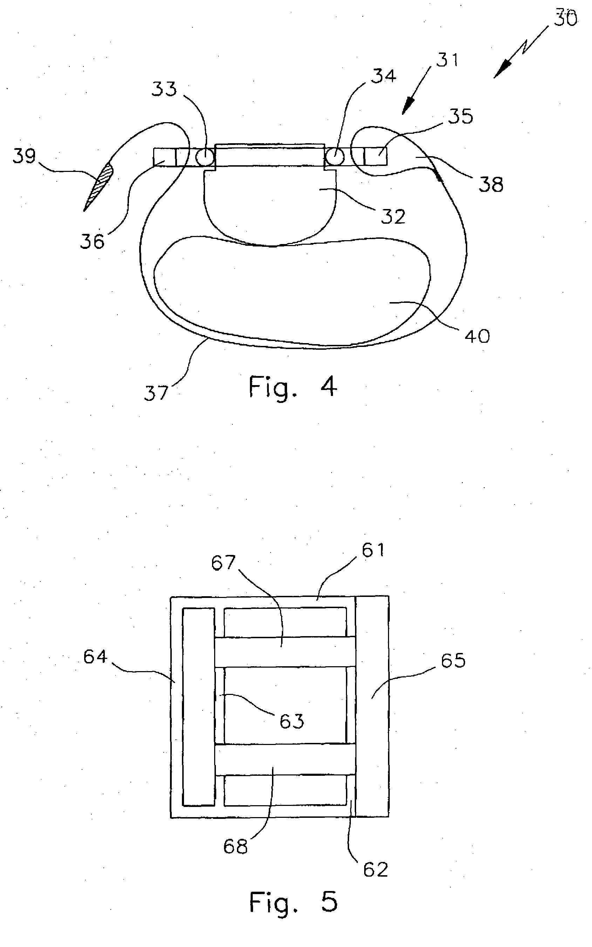

Die Schnalle 31 weist auch äußere Stege 35 und 36 auf, um die herum ein Verband 37 geführt wird (siehe Fig. 4). Der Verband 37 weist im Bereich des Steges 35 eine vernähte Schlaufe 38 auf. Von dort wird der Verband 37 um ein Körperteil 40, welches das Handgelenk sein kann, herumgeführt. Im Bereich des Steges 36 wird der Verband 37 mit seinem offenen Ende durch die Schnalle 31 hindurchgeführt und zum Spannen nach unten gezogen. Dadurch wird das Kompressorium 32 gegen das Körperteil 40 und somit gegen die zuzudrückende Wunde gepresst. Ein Verschluss 39, der ein Klettverschluss oder ein Schnappverschluss sein kann und im Bereich des freien Endes des Verbandes 37 angeordnet ist, kann nach dem Positionieren und Spannen des Verbandes 37 an den Verband 37 angedrückt werden, um die Position des Verbandes 37 und seine Spannung zu halten.

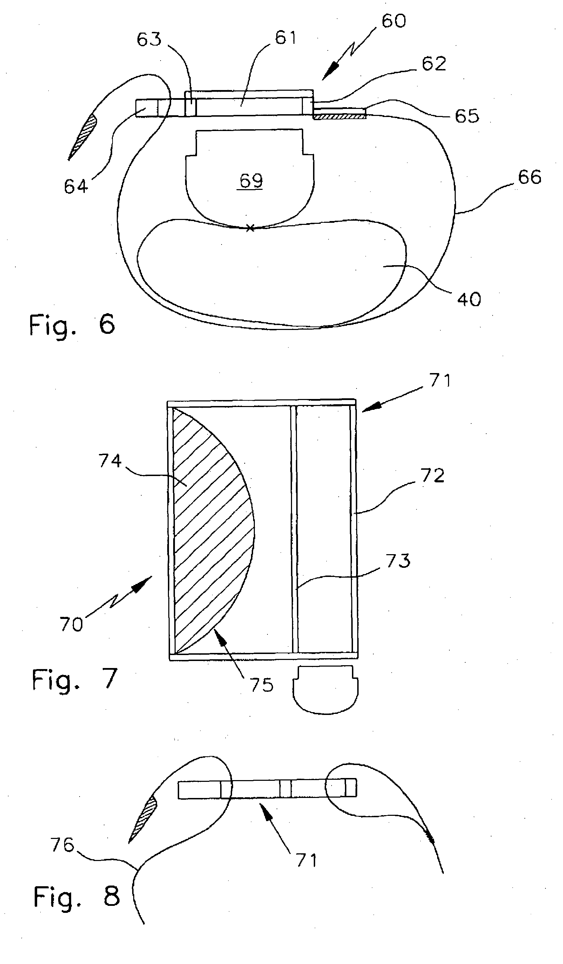

Die Fign. 5 und 6 zeigen eine Einrichtung 60 mit einer Schnalle 61, die Stege 62, 63 und 64 aufweist. An dem äußeren Steg 62 ist eine Platte 65 angebracht, an welcher ein Verband 66 befestigt ist, vorzugsweise durch Verklebung oder Vernähung. Ferner sind die Stege 62 und 63 mit Bügeln 66 und 67 verbunden. Die Bügel 67 und 68 verhindern, dass ein Kompressorium 69 nach oben heraus gedrückt werden kann.

Eine Einrichtung 70 zeigen die Fign. 7 und 8. Die Einrichtung 70 weist eine Schnalle 71 mit Stegen 72, 73 und 74 auf. Der Steg 74 weist eine konvexe Wölbung 75 auf, über welche ein Verband 76 herumgeführt wird (siehe Fig. 8). Die Wölbung 75 sorgt dafür, dass der Verband 76 entlang seiner Breite gestreckt bleibt und sich nicht entlang seiner Breite wurstartig zusammenschieben kann.

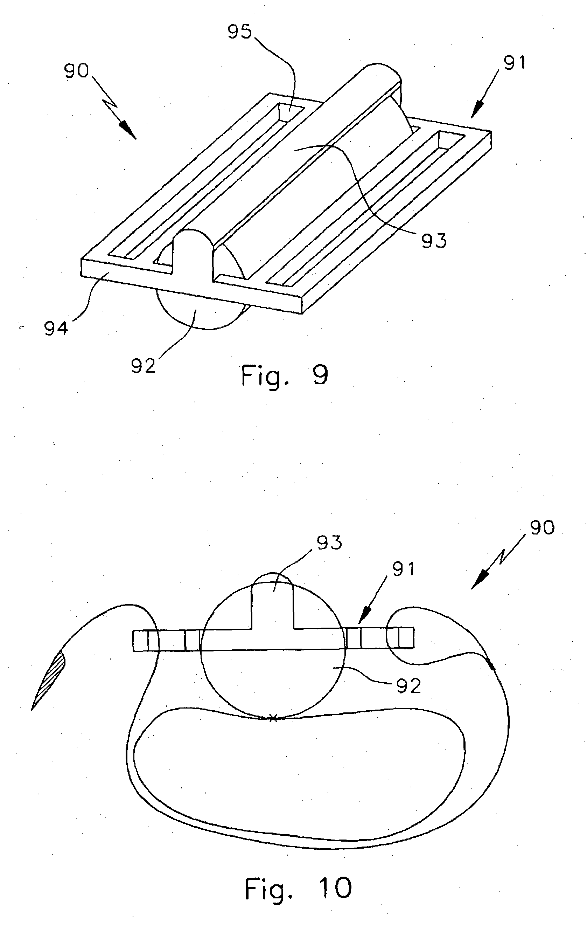

Die Fign. 9 und 10 zeigen eine Einrichtung 90 mit einer Schnalle 91 und einem Kompressorium 92. Die Schnalle 91 weist einen Bügel 93 auf, der Querstege 94 und 95 miteinander verbindet. Der Bügel 93 ragt über die Schnalle 91 raus und bildet somit nach oben einen Anschlag für das Kompressorium 92, so dass das Kompressorium 92 nicht nach oben herausgedrückt werden kann.

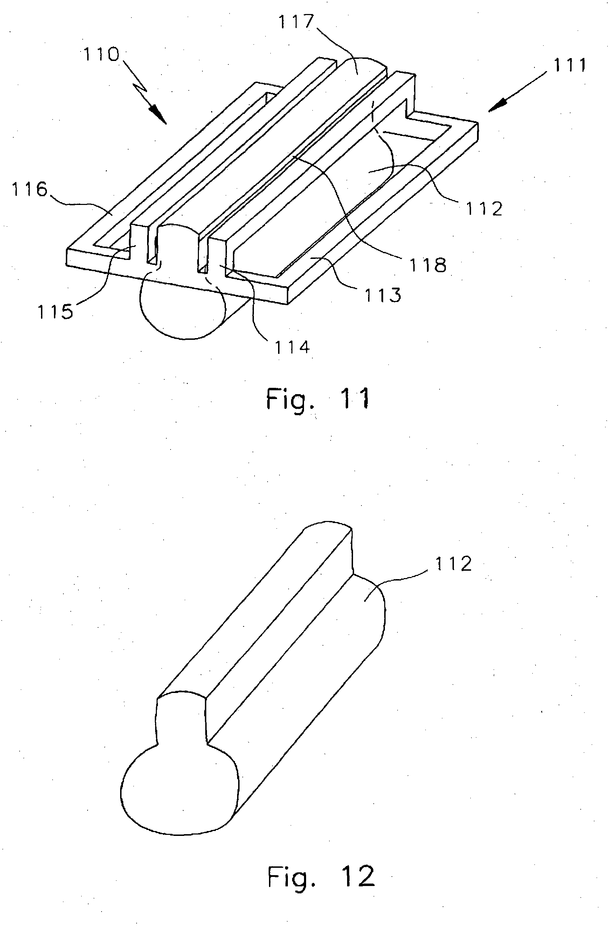

Eine Einrichtung 110 ist mit einer Schnalle 111 und einem Kompressorium 112 ausgerüstet (siehe Fign. 11 bis 13). Die Schnalle 111 weist Stege 113, 114, 115 und 116 auf. Die Stege 114 und 115 stehen nach oben über die Schnalle 111 heraus. Der Verband 37 kann somit über die Stege 114 und 115 herumgeführt werden. Das freie Ende des Verbandes 37, das über den Steg 115 geführt wird, kann aufgrund der Höhe des Steges 115 besonders gut gespannt werden. Ein Bügel 117, der parallel zu den Stegen 114 und 115 angeordnet ist verhindert, dass das Kompressorium 112 nach oben heraus gedrückt werden kann. Damit das Kompressorium 112 nicht um seine Längsachse verschwenkt werden kann, sondern stabil die dargestellte Position einnimmt, weist der Bügel 117 auf beiden Seiten einen nach unten gezogenen Rand 118 auf.

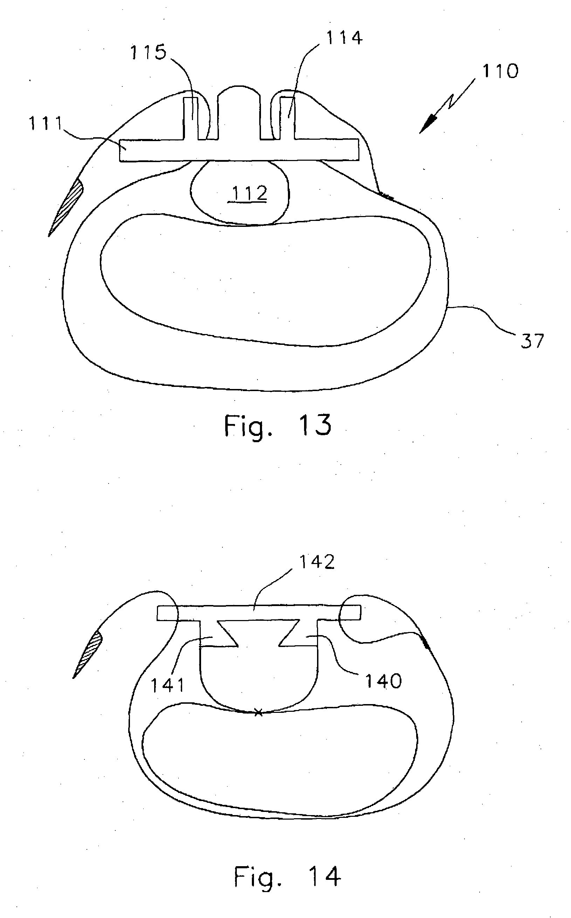

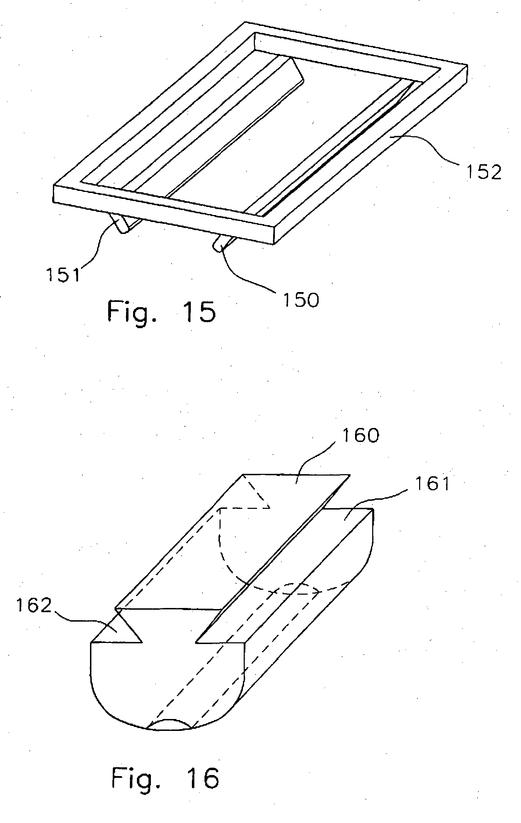

Fig. 16 zeigt ein Kompressorium 160 mit schwalbenschwanzartigen Aussparungen 161 und 162. In die schwalbenschwanzartigen Aussparungen 161 und 162 können Stege 140 und 141 einer Schnalle 142 (siehe Fig. 14) und Stege 150 und 151 einer Schnalle 152 (siehe Fig. 15) eingreifen.

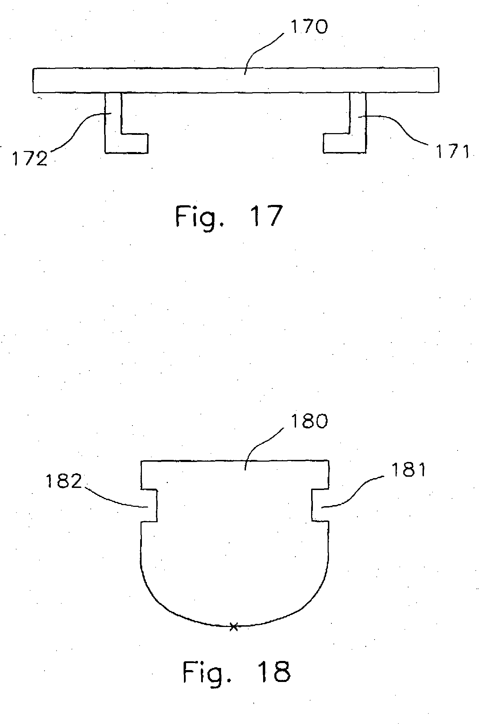

Fig. 17 zeigt eine Schnalle 170, die mit einem Kompressorium 180 (siehe Fig. 18) formschlüssig verbunden werden kann. Die Schnalle 170 ist aus diesem Grund mit Stegen 171 und 172 ausgestaltet, die mit abgebogenen und aufeinander zuweisenden Enden versehen sind. Das Kompressorium 180 ist mit Aussparungen 181 und 182 versehen, in welche die abgebogenen und aufeinander zu weisenden Enden der Stege 171 und 172 eingreifen können.

**BEZUGSZEICHENLISTE**

| | | | |
|---|---|---|---|
| 10 | Schnalle | 62 | Steg |
| 11 | Steg | 63 | Steg |
| 12 | Steg | 64 | Steg |
| 13 | Steg | 65 | Platte |
| 14 | Steg | 66 | Verband |
| 15 | Quersteg | 67 | Bügel |
| 16 | Quersteg | 68 | Bügel |
| | | 69 | Kompressorium |
| 20 | Einrichtung | | |
| 21 | Kompressorium | 70 | Einrichtung |
| 22 | Aussparung | 71 | Schnalle |
| 23 | Aussparung | 72 | Steg |
| 24 | Lupe | 73 | Steg |
| 25 | Bügel | 74 | Steg |
| | | 75 | Wölbung |
| 30 | Einrichtung | 76 | Verband |
| 31 | Schnalle | | |
| 32 | Kompressorium | 90 | Einrichtung |
| 33 | Scharniere | 91 | Schnalle |
| 34 | Scharniere | 92 | Kompressorium |
| 35 | Steg | 93 | Bügel |
| 36 | Steg | 94 | Quersteg |
| 37 | Verband | 95 | Quersteg |
| 38 | Schlaufe | | |
| 39 | Klettverschluss | 110 | Einrichtung |
| 40 | Körperteil | 111 | Schnalle |
| | | 112 | Kompressorium |
| 60 | Einrichtung | 113 | Steg |
| 61 | Schnalle | 114 | Steg |
| 115 | Steg | | |
| 116 | Steg | 160 | Kompressorium |
| 117 | Bügel | 161 | Aussparung |
| 118 | Rand | 162 | Aussparung |
| | | | |
| 140 | Steg | 170 | Schnalle |
| 141 | Steg | 171 | Steg |
| 142 | Schnalle | 172 | Steg |
| | | | |
| 150 | Steg | 180 | Kompressorium |
| 151 | Steg | 181 | Aussparung |
| 152 | Schnalle | 182 | Aussparung |

## Patentansprüche

1. Einrichtung (20, 30, 60, 70, 90, 110) zum Andrücken eines Kompressoriums (21, 32, 69, 92, 112, 160, 180) mit einer Schnalle (10, 31, 61, 71, 91, 111, 142, 152, 170), durch die ein Verband (37, 66, 76) führbar ist, wobei die Schnalle (10, 31, 61, 71, 91, 111, 142, 152, 170) mindestens drei Stege (11, 12, 13, 14, 35, 36, 62, 63, 64, 72, 73, 74, 113, 114, 115, 116, 140, 141, 150, 151, 171, 172) aufweist, die zwei einander gegenüberliegende Querstege (15, 16, 94, 95) miteinander verbinden, **dadurch gekennzeichnet, dass** das Kompressorium (21, 32, 69, 92, 112, 160, 180) zwischen zwei benachbarten Stegen (12, 13, 33, 34, 62, 63, 72, 73, 114, 115, 140, 141, 150, 151, 171, 172) anordenbar ist und die Einrichtung (20, 30, 60, 70, 90, 110) mindestens einen einen Anschlag nach oben bildenden über die Schnalle (61, 91, 111) herausragenden Bügel (25, 67, 68, 93, 117) für das Kompressorium (21, 32, 69, 92, 112, 160, 180) aufweist.

2. Einrichtung (30) nach Anspruch 1, **dadurch gekennzeichnet, dass** die Schnalle (31) an den Querstegen mindestens ein Scharnier (33, 34), aufweist.

3. Einrichtung (60) nach Anspruch 1 oder 2, **dadurch gekennzeichnet, dass** die Schnalle (61) an einem der beiden äußeren Stege (62) eine Platte (65) aufweist.

4. Einrichtung (70) nach einem der Ansprüche 1 bis 3, **dadurch gekennzeichnet, dass** einer der äußeren Stege (74) eine der Schnalleninnenseite zugewandte konvexe Wölbung (75) aufweist.

5. Einrichtung (20) nach einem der Ansprüche 1 bis 4, **dadurch gekennzeichnet, dass** das Kompressorium (21) als eine Lupe ausgebildet ist oder eine Lupe (24) aufweist.

6. Einrichtung (20, 30, 60, 70, 90, 110) nach einem der Ansprüche 1 bis 5, **dadurch gekennzeichnet, dass** das Kompressorium (21, 32, 69, 92, 112, 160, 180) aus einem elastisch verformbaren Material hergestellt ist.

7. Verband (37, 66, 76), **dadurch gekennzeichnet, dass** er die Einrichtung (20, 30, 60, 70, 90, 110) nach einem der Ansprüche 1 bis 6 aufweist.

8. Verband (37, 66, 76) nach Anspruch 7, **dadurch gekennzeichnet, dass** er einenends eine geschlossene Schlaufe (38) aufweist, die um einen der mindestens drei Stege (11, 12, 14, 35, 36, 62, 63, 64, 72, 73, 74, 113, 114, 115, 116, 140, 141, 150, 151, 171, 172) herumgeführt ist, und er anderenends um einen weiteren der mindestens drei Stege (11, 12, 14, 35, 36, 62, 63, 64, 72, 73, 74, 113, 114, 115, 116, 140, 141, 150, 151, 171, 172) herumgeführt ist und einen Verschluss (39) aufweist.

9. Verband (37, 66, 76) : nach Anspruch 7 oder 8, **dadurch gekennzeichnet, dass** der Verband (37, 66, 76) einstückig ist.

## Claims

1. A device (20, 30, 60, 70, 90, 110) for pressing on a compress (21, 32, 69, 92, 112, 160, 180) with a clasp (10, 31, 61, 71, 91, 111, 142, 152, 170), through which a dressing (37, 66, 76) can be passed, wherein the clasp (10, 31, 61, 71, 91, 111, 142, 152, 170) has at least three bars (11, 12, 13, 14, 35, 36, 62, 63, 64, 72, 73, 74, 113, 114, 115, 116, 140, 141, 150, 151, 171, 172) which connect two opposing cross struts (15 16, 94, 95) to each other, **characterised in that** the compress (21, 32, 69, 92, 112, 160, 180) can be arranged between two adjacent bars (12, 13, 33, 34, 62, 63, 72, 73, 114, 115, 140, 141, 150, 151, 171, 172) and the device (20, 30, 60, 70, 90, 110) has, projecting out upwards over the clasp (61, 91, 111) and forming a stop,_at least one bracket (25, 67, 68, 93, 117) for the compress (21, 32, 69, 92, 112, 160, 180).

2. The device (30) according to claim 1, **characterised in that** the clasp (31) has a least one hinge (33, 34) on the cross struts.

3. The device (60) according to claim 1 or 2, **characterised in that** on one of the two outer bars (62) the clasp (61) has a plate (65).

4. The device (70) according to any one of claims 1 to 3, **characterised in that** one of:the outer bars (74) has a convex curvature (75) facing the inside of the clasp.

5. The device (20) according to any one of claims 1 to 4, **characterised in that** the compress (21) is designed as a magnifying glass or has a magnifying glass (24).

6. The device (20, 30, 60, 70, 90, 110) according to any one of claims 1 to 5, **characterised in that** the compress (21, 32, 69, 92, 112, 160, 180) is made of an elastically deformable material.

7. A dressing (37, 66, 75) **characterised in that** it has the device (20, 30, 60, 70, 90, 110) according to any one of claims 1 to 6.

8. The dressing (37, 66, 76) according to claim 7, **characterised in that** at one end it has a closed loop (38) which is passed around one of the at least three bars (11, 12, 14, 35, 36, 62,: 63, 64, 72, 73, 74, 113, 114, 115, 116, 140, 141, 150, 151, 171, 172) and at the other end it is passed around a further one of the at least three bars (11, 12, 14, 35, 36, 62, 63, 64, 72, 73, 74, 113, 114, 115, 116, 140, 141, 150, 151, 171, 172) and has a closure:(39).

9. The dressing (37, 66, 76) according to claim 7 or 8, **characterised in that** the pressing (37, 66, 75) is produced in one piece.:

## Revendications

1. Système (20, 30, 60, 70, 90, 110) destiné à presser un dispositif de compression (21, 32, 69, 92, 112, 160, 180) avec une boucle (10, 31, 61, 71, 91, 111, 142, 152, 170) à travers laquelle on peut faire passer un bandage (37, 66, 76), la boucle (10, 31, 61, 71, 91, 111, 142, 152, 170) comportant au moins trois barrettes (11, 12, 13, 14, 35, 36, 62, 63, 64, 72, 73, 74, 113, 114, 115, 116, 140, 141, 150, 151, 171, 172) reliant ensemble deux barrettes latérales (15, 16, 94, 95) mutuellement opposées, **caractérisé en ce que** le dispositif de compression (21, 32, 69, 92, 112, 160, 180), peut se: placer entre deux barrettes (12, 13, 33, 34, 62, 63, 72, 73, 114, 115, 140, 141, 150, 151, 171, 172) voisines et l'installation (20, 30, 60, 70, 90, 110) comportant au moins un étrier (25, 67, 68, 93, 117) saillant par-dessus la boucle (61, 91, 111), formant une butée vers le haut pour le dispositif de compression (21, 32, 69, 92, 112, 160, 180).

2. Système (30) selon la revendication 1, **caractérisé en ce que** la boucle (31) comporte au moins une charnière (33, 34) sur les barrettes latérales.

3. Système (30) selon la revendication 1 ou 2, **caractérisé en ce que** sur l'une: des deux barrettes (62) extérieures, la boucle (61) comporte une plaque (65).

4. Système (70) selon l'une quelconque des revendications 1 à 3, **caractérisé en ce que** l'une des barrettes (74) extérieures comporte une voûte (75) convexe dirigée vers la face intérieure de la boucle.

5. Système (20) selon l'une quelconque des revendications 1 à 4, **caractérisé en ce que** le dispositif de compression (21) est conçu en tant que loupe ou comporte une loupe (24).

6. Système (20, 30, 60, 70, 90, 110) selon l'une quelconque des revendications 1 à 5, **caractérisé en ce que** le dispositif de compression (21, 32, 69, 92, 112, 160, 180) est fabriqué dans une matière élastiquement déformable.

7. Bandage (37, 66, 76), **caractérisé en ce qu'**il comporte le système (20, 30, 60, 70, 90, 110) selon l'une quelconque des revendications 1 à 6.

8. Bandage (37, 66, 76) selon la revendication 7, **caractérisé en ce qu'**il comporte sur une extrémité un passant (38) fermé qui est guidé autour de l'une des au moins trois barrettes (11, 12, 13, 14, 35, 36, 62, 63, 64, 72, 73, 74, 113, 114, 115, 116, 140, 141, 150, 151, 171, 172) et que sur l'autre extrémité, il est guidé autour d'une autre barrette des au moins trois barrettes (11, 12, 13, 14, 35, 36, 62, 63, 64, 72, 73, 74, 113, 114, 115, 116, 140, 141, 150, 151, 171, 172) et comporte une fermeture (39).

9. Bandage (37, 66, 76) selon la revendication 7 ou 8, **caractérisé en ce que** le bandage (37, 66, 76) est en une seule partie.
